# FASCICULE DE BREVET EUROPEEN

(11) **EP 3 737 234 B1**
(45) Date de publication et mention de la délivrance du brevet: **23.08.2023**
(21) Numéro de dépôt: 19700261.1
(22) Date de dépôt: 08.01.2019
(51) Int. Cl.: A01N 25/10, A01P 7/04, A01P 17/00, C08J 3/20, A61L 9/04, A61Q 15/00, A61Q 13/00, A61K 8/72

(54) **MATÉRIAU POLYMÉRIQUE SOLIDE IMPRÉGNÉ D'UNE SUBSTANCE ORGANIQUE VOLATILE ET D'UN ESTER PARTICULIER ET SES UTILISATIONS**
FESTES POLYMERES MATERIAL IMPRÄGNIERT MIT EINER FLÜCHTIGEN ORGANISCHEN SUBSTANZ UND EINEM SPEZIFISCHEN ESTER UND VERWENDUNGEN DAVON
SOLID POLYMERIC MATERIAL IMPREGNATED WITH A VOLATILE ORGANIC SUBSTANCE AND A SPECIFIC ESTER AND USES OF SAME

(30) Priorité: 11.01.2018 FR 1850218
(43) Date de publication de la demande: 18.11.2020
(73) Titulaire: Jafer Enterprises R&D SL, 08403 Granollers (ES)
(72) Inventeur: DELMAS, Thomas, 06600 ANTIBES (FR); PEREZ, Marion, 06270 VILLENEUVE-LOUBET (FR); LE BRAS, Marine, 06600 ANTIBES (FR); GOUTEYRON, Antoine, 06110 LE CANNET (FR)
(74) Mandataire: Renard, Emmanuelle
(86) Numéro de dépôt international: PCT/EP2019/050303
(87) Numéro de publication internationale: WO 2019/137894

(56) Documents cités:
- WO-A1-02/47735
- WO-A1-2018/122209
- US-A1- 2015 020 915

## Description

### DOMAINE DE L'INVENTION

La présente invention a trait au domaine des articles utilisés pour parfumer ou désodoriser l'atmosphère ou le linge ou comme répulsifs d'insectes ou insecticides, qui comprennent un matériau polymérique solide imprégné d'une substance organique volatile.

### ARRIERE-PLAN DE L'INVENTION

Les articles disponibles dans le commerce comme diffuseurs solides de parfums ou d'insecticides peuvent prendre différentes formes adaptées à leur utilisation. On citera par exemple les bracelets anti-moustiques ou les diffuseurs sous forme de galets à poser ou à suspendre pour parfumer le linge ou les voitures. Ces produits sont généralement constitués d'une matrice organique (notamment polymérique) ou inorganique (telle que de l'argile) dans laquelle le parfum ou l'insecticide a été introduit par imprégnation ou parfois, pour les matrices polymères, avant polymérisation de cette dernière.

L'inconvénient de ces produits est que la substance volatile qu'ils renferment a tendance à s'évaporer rapidement au départ et est ensuite libérée en quantité insuffisante, ce qui nuit à l'efficacité de ces diffuseurs dans le temps.

Pour remédier à cet inconvénient, il a été proposé d'associer la substance volatile à un régulateur de diffusion. Le brevet FR 1 601 586 décrit ainsi des matériaux cellulaires, préférentiellement à base de cellulose, imprégnés de substances volatiles, plus particulièrement d'un insecticide, dans lesquels la vitesse de diffusion de l'insecticide est régulée par ajout d'un composé permettant d'abaisser sa tension de vapeur, choisi notamment parmi certains diesters d'alkyle. Ces composés sont de préférence associés à des huiles de silicone qui contribuent à la vaporisation contrôlée de l'insecticide. La Demanderesse a toutefois démontré que les composés précités ne permettaient pas d'atteindre un taux suffisant de substances volatiles imprégnées dans le matériau, ni un profil de diffusion satisfaisant de ces substances, en particulier dans le cas de matériaux siliconés.

Par ailleurs, le brevet US-6,379,689 décrit des diffuseurs comprenant une matrice à base d'élastomère de silicone dans laquelle sont dispersés une substance volatile, telle qu'un parfum ou un insecticide, ainsi qu'un agent destiné à améliorer la compatibilité de la substance volatile avec la matrice, qui est choisi parmi des éthers tels que le 3-méthyl-3-méthoxybutanol. La substance volatile et l'agent compatibilisant sont mélangés à la résine siliconée liquide qui est ensuite réticulée. Ils se trouvent donc piégés dans la matrice siliconée lors de sa synthèse. Il a été observé par la Demanderesse que ces agents compatibilisants présentaient les mêmes limitations que les solvants décrits dans FR 1 601 586. Par ailleurs, ces agents compatibilisants ne permettent pas de limiter les phénomènes d'exsudation de la substance volatile au cours du stockage, qui doit être contrée par ajout d'un tensioactif au mélange à polymériser. Enfin, le procédé de fabrication des diffuseurs divulgués dans le document US-6,379,689 est complexe, ce qui impacte nécessairement le prix de revient de ces derniers.

Il subsiste donc le besoin de disposer d'un matériau polymérique solide capable de diffuser de manière prolongée une substance organique volatile, qui puisse être introduite dans le matériau en quantité suffisante par simple imprégnation.

Il serait également utile de disposer d'un article en matériau polymérique solide à base de résine de silicone qui puisse être imprégné d'une quantité suffisante de substance organique volatile sans subir de déformation, notamment de gonflement.

Il a été proposé dans le document WO2018/122209 (document de l'état de l'art selon l'article 54(3) CBE) des déodorants qui se présentent de préférence sous forme d'aérosols et en variante sous forme de sticks comprenant un élastomère de silicone et un parfum. Dans ces produits, le parfum est mélangé aux autres constituants qui sont ensuite coulés sous forme de stick et n'est pas imprégné dans une matrice polymérique solide.

### RESUME DE L'INVENTION

La Demanderesse a démontré que les besoins précités pouvaient être satisfaits en imprégnant un matériau polymérique solide d'une solution renfermant en plus d'une substance organique volatile (telle qu'un parfum) un ester particulier qui permet d'améliorer le taux d'infusion de la substance organique volatile dans le matériau et son profil de diffusion.

L'invention a ainsi pour objet un matériau polymérique solide imprégné d'une substance organique volatile et d'un solvant comprenant un monoester en C₁₀-C₁₈ d'alkyle en Ci-Cs selon la revendication 1.

Elle a également pour objet un procédé de fabrication de ce matériau, caractérisé en ce qu'il comprend les étapes suivantes :
(a) la solubilisation de la substance organique volatile dans le solvant, et
(b) l'imprégnation du matériau polymérique solide avec la solution ainsi obtenue.

L'invention a encore pour objet l'utilisation non thérapeutique du matériau précité pour parfumer ou désodoriser l'atmosphère, le corps ou le linge ou comme répulsif d'insectes ou insecticide, de préférence pour parfumer l'atmosphère.

Elle a en outre pour objet un article comprenant ce matériau, caractérisé en ce qu'il est choisi parmi un produit parfumant ou désodorisant, un répulsif d'insectes ou un insecticide, de préférence un produit parfumant.

### DESCRIPTION DETAILLEE

L'invention concerne un matériau polymérique solide. Par "solide", on entend que la forme et les dimensions du matériau ne varient pas à température ambiante, en l'absence de force appliquée au matériau.

Ce matériau polymérique peut comprendre un polymère thermoplastique ou thermodurcissable ou d'un élastomère, pour autant que le matériau présente une perméabilité suffisante aux gaz et qu'il ne soit pas soluble dans les solvants décrits ci-après. Il s'agit avantageusement d'un élastomère. L'élastomère peut être obtenu par réticulation d'un ou plusieurs précurseurs liquides organiques ou organominéraux, choisis parmi les résines de silicone, les fluoroélastomères, les perfluoroélastomères, le caoutchouc naturel, le polysisoprène synthétique, le polybutadiène, les copolymères styrène-butadiène, le polyisobutylène (PIB), le chloroprène, les copolymères butadiène-acrylonitrile, les copolymères éthylène-propylène (EP ou EPM), les terpolymères éthylène-propylène-diène (EPDM), les polyéther bloc amides (PEBA), les polymères blocs du type EPDM-polypropylène, les polyuréthanes thermoplastiques (TPU), les oléfines thermoplastiques (TPO), les polysulfures, les copolymères éthylène-acétate de vinyle (EVA ou EVM), les élastomères polyacryliques (ACM), les copolymères éthylène acrylique (AEM), le polyéthylène chlorosulfoné (CSM), les élastomères d'épichlorhydrine (CO et ECO) et leurs mélanges.

On préfère que le matériau polymérique selon l'invention comprenne un élastomère de silicone obtenu à partir d'au moins une résine de silicone, seul ou en mélange avec au moins un autre polymère. L'élastomère de silicone est généralement obtenu par réaction d'un organopolysiloxane avec soit un peroxyde organique, soit un agent réticulant en présence d'un catalyseur. L'agent réticulant peut être, dans le cas d'une résine bicomposant, un polysiloxane renfermant au moins un groupe réactif (hydrogène, allyle ou vinyle, notamment) capable de réagir avec un groupe réactif porté par l'organopolysiloxane (qui est par exemple un organohydrogenpolysiloxane). Il est bien entendu que les groupes réactifs précités peuvent être situés en position terminale et/ou latérale de la chaîne de polysiloxane. L'organopolysiloxane formant le précurseur liquide et l'agent réticulant ont chacun en principe un squelette polydiméthylsiloxane dont un ou plusieurs groupes méthyle ont été substitués ou non par des groupes phényle. Dans le cas d'une résine monocomposant, l'organopolysiloxane comprend généralement une extrémité silanol et l'agent réticulant peut être un alkyltrialcoxysilane tel que le méthyltriacétoxysilane. Dans tous les cas, le catalyseur est généralement à base d'étain et/ou de titane.

Le matériau polymérique peut également renfermer un ou plusieurs additifs habituellement utilisés dans de tels matériaux, choisis parmi : des charges organiques et/ou inorganiques ; des renforts ; des plastifiants ; des pigments et/ou colorants ; des anti-oxydants ; des retardateurs de flamme ; des absorbeurs UV ; des stabilisants à la lumière ; des agents anti-chocs ; des agents antistatiques ; des fongicides ; et leurs mélanges.

Ce matériau peut par exemple présenter une dureté Shore A allant de 10 à 100, préférentiellement de 20 à 80.

En tout état de cause, l'homme du métier saura choisir le matériau adapté à la diffusion de la substance organique volatile choisie, de telle manière que la seconde ne dégrade pas le premier dans les conditions normales d'utilisation.

Le matériau polymérique solide est imprégné d'une substance organique volatile. Par « substance organique volatile », on entend un produit constitué d'une ou plusieurs molécules organiques, qui possède une tension de vapeur supérieure à la pression atmosphérique à température ambiante. La substance organique volatile utilisée selon l'invention peut avantageusement être choisie parmi un parfum, un agent masqueur d'odeur et un insecticide, de préférence un parfum.

Dans le cadre de cette description, on entend par "parfum" un composé unique ou un mélange de composés volatils et odoriférants. Ces composés sont notamment listés dans Merck Index, 8th Edition, Merck & Co., Inc. Rahway, N.J. Ces composés peuvent être d'origine synthétique ou naturelle. Il peut par exemple s'agir d'une ou plusieurs huiles essentielles de plantes, choisies par exemple parmi les Astéracées, les Myrtacées, les Lauracées, les Lamiacées, les Rutacées et les Zingibéracées, qui sont habituellement extraites d'une partie quelconque de ces plantes par extraction à l'aide d'un fluide supercritique, hydrodistillation, enfleurage, entraînement à la vapeur ou tout autre procédé permettant l'extraction de molécules parfumées d'une plante.

Qu'ils soient d'origine synthétique ou naturelle, les parfums comprennent en général des composés, généralement terpéniques ou aromatiques, choisis parmi les hydrocarbures, les alcools et leurs esters, les aldéhydes, les esters, les acétals et les cétones, ainsi que les composés macrocycliques en C₁₂-C₁₆, les composés hétérocycliques tels que les pyrazines et les indoles, et leurs mélanges.

Les composés parfumants suivants peuvent notamment être utilisés comme parfum dans la présente invention, seuls ou en combinaison : le 2-méthyl butyrate de méthyle ; le 2-méthyl butyrate d'isopropyle ; le 2-méthyl butyrate d'éthyle ; le 2-méthyl pentanoate d'éthyle ; l'heptanoate d'éthyle ; l'octanoate d'éthyle ; l'hexanoate d'isobutyle ; le butyrate d'amyle ; l'heptanoate d'amyle ; l'isobutyrate d'isoamyle ; l'acétate d'hexyle ; le butyrate d'hexyle ; l'isobutyrate d'hexyle ; l'isovalérate d'hexyle ; le propionate d'hexyle ; le 2-cyclohexyl propanoate d'éthyle ; le 3,5,5-triméthyl hexanoate d'éthyle ; le 5-hydroxydécanoate de glycéryle ; l'acétate de prényle ; le 2-butenyl acétate de méthyle ; le 3-nonénoate de méthyle ; le décénoate d'éthyle ; l'octénoate d'éthyle ; le décadiénoate d'éthyle ; l'octénoate d'éthyle ; l'acétate de citronellyle ; l'isovalerate de 2-hex-1-ènyle; le propionate de 2-hexèn-1-yle ; le valérate de 2-hexènen-1-yle ; le (E)-2-hexènoate de 3-hexèn-1-yle ; le 2-méthyl butyrate de 3-hexèn-1-yle ; l'acétate de 3-hexèn-1-yle ; le benzoate de 3-hexèn-1-yle ; le formate de 3-hexèn-1-yle ; le tiglate de 3-hexèn-1-yle ; le 2-méthyl butyrate de 2-méthyl butyle ; l'isovalérate de butyle ; l'allyl cyclohexane ; le propionate d'allyl cyclohexyle ; le valérate d'allyl cyclohexyle ; l'octanoate de benzyle ; la gamma-décalactone ; la gamma-dodécalactone ; la jasmin lactone ; la jasmolactone ; la nonalactone ; le 6-acétoxydihydrothéaspirane ; l'isobutyrate de phénoxyéthyle ; le pivacyclène ; l'anthranilate de diméthyle ; l'anthranilate de méthyle ; l'octanal ; le nonanal ; le décanal ; le dodécanal ; le méthyl nonyl acétaldéhyde ; le méthyl octyl acétaldéhyde ; le 2,4-hexadiénal ; l'intreleven ; le décèn-1-al ; le nonèn-1-al ; l'aldoxal ; le géraldéhyde ; l'isocyclocitral ; le d-limonène ; le ligustral ; le tridécénal ; le triplal ; le vertoliff ; le cyclal C; l'héliotropine ; le néocaspirène ; le beta naphthol éthyl éther ; le beta naphthol méthyl éther ; le hyacinth éther ; la 2-heptyl cyclopentanone ; l'undecavertol ; le frutonile ; et leurs mélanges.

En variante, la substance organique volatile selon l'invention peut constituer un agent masqueur d'odeur. De tels agents peuvent notamment être choisis parmi : les aldéhydes saturés éventuellement alcoxylés, tels que le 2-méthylundécanal et le 6-méthoxy-2,6-diméthyloctanal ; les aldéhydes insaturés, tels que 2,6-diméthylhept-5-énal, le 2,4-diméthylcyclohex-3-énecarbaldéhyde, le 4-vinylcyclohex-1-ènecarbaldéhyde et le bicyclo[2.2.2]oct-5-ène-2-carboxaldéhyde ; les aldéhydes aromatiques, tels que le 4-méthylbenzaldéhyde ; les glycolates, tels que l'allyl-2-(isopentyloxy)acétate ; les alcools, tels que le linalool, le nona-2,6-dién-1-ol, le non-6-èn-1-ol, le 2,6-diméthylheptan-2-ol et le *cis* hex-3-èn-1-ol ; les parfums soufrés tels que le 2-(4-méthylcyclohex-3-èn-1-yl)propane-2-thiol et le 4,7,7-triméthyl-6-thiabicyclo([3.2.1]octane ; les benzodioxépines, telles que la 7-méthyl-2H-benzo[b][1,4]dioxépin-3-4H)-one ; les cétones, telles que la 3-méthyl-2-pentylcyclopent-2-énone et la 2-méthyl-5-(prop-1-èn2-yl)cyclohex-2-énone ; les furanes, tels que le 5-tert-butyl-2-méthyl-5-propyl-2H-furane ; les pyranes, tels que le 4-méthylène-2-phényltétrahydro-2H-pyrane ; les phénols, tels que l'eugénol (4-allyl-2-méthoxyphénol) ; les esters phénoliques, tels que l'acétate de p-tolyle ; les nitriles, tels que le 4-isopropyl benzonitrile ; les éthers aromatiques, tels que le 1-méthoxy-4-propylbenzène ; les esters, tels que l'isobutyrate d'éthyle, l'acétate d'hexényle et l'isovalérate d'éthyle ; les lactones, telles que la 5-pentyldihydrofuran-2(3H)-one ; les anthranilates, tels que le 2-aminobenzoate de méthyle ; les acides carboxyliques, tels que l'acide 2-phénylacétique ; les terpènes, tels que le 7-méthyl-3-méthylèneocta-1,6-diène ; et leurs mélanges.

Un autre exemple d'agent masqueur d'odeur est constitué d'une combinaison de 1,8-para-menthènethiol et d'acétate de 3-mercaptohexyle ou d'une huile essentielle de Timur *(Zanthoxylum armatum*) contenant cette combinaison, qui peut être obtenue à partir de toute partie du Timur et notamment de ses feuilles, de son tronc, de ses fruits (baies) ou de leur péricarpe, plus préférentiellement de ses baies.

En variante encore, la substance organique volatile selon l'invention peut constituer un insecticide. Des exemples d'insecticides comprennent notamment : les huiles essentielles telles que celles de patchouli, de menthe poivrée, de cyprès, de pin, de Perilla, de pyrèthre *(Tanacetum cinerariifolium*), de cataire, de lavande, de coriandre, d'eucalyptus, de citronnelle, de citron vert, de néroli, de romarin, d'hysope, de rose, d'ylang-ylang, de poivre, de cannelle, de camphre, de camomille, de laurier, d'estragon, d'absinthe, de trèfle, de géranium, de sauge, de basilic, de persil, d'anis étoilé, de fenouil, de galbanum, de manuka, de violette, d'aneth, d'angélique, de curcuma, de gingembre, d'ambrette et de gaulthérie ; les composés de synthèse tels que le N,N'-diéthyl-3-méthylbenzamide (DEET), le p-menthane-3,8-diol, la 2-undécanone, le 2-(2-hydroxyéthyl)-1-méthylpropylester d'acide 1-pipéridine carboxylique et le butylacétylaminopropionate d'éthyle (IR 3535) ; et leurs mélanges.

La substance organique volatile est solubilisée dans un solvant particulier avant d'être introduite dans le matériau polymérique solide décrit précédemment. En tout état de cause, l'homme du métier saura choisir une substance organique volatile soluble en quantité suffisante pour l'application recherchée dans le solvant décrit ci-après.

Ce solvant comprend un monoester en C₁₀-C₁₈ d'alkyle en C₁-C₈, tel que le laurate de méthyle, le laurate d'isopropyle, le laurate d'éthylhexyle, le myristate d'isopropyle, le stéarate de butyle, le stéarate d'isobutyle, le palmitate d'éthyle ou le palmitate d'éthylhexyle, sans que cette liste ne soit limitative. On utilise de préférence un monoester en C₁₂-C₁₆ d'alkyle en C₃-C₆, plus préférentiellement le myristate d'isopropyle.

Dans le cas où le matériau polymérique solide comprend un élastomère de silicone, on préfère que le solvant renferme en outre un diester saturé en C₂-C₁₀ d'alkyle en C₃-C₁₂, tel que le succinate de diméthyle, le succinate de diéthyle, de succinate de diéthylhexyle, le glutarate de diméthyle, l'adipate de diméthyle, l'adipate de diisopropyle, l'adipate de diisobutyle, l'adipate d'octyle, le sébaçate de diméthyle, le sébaçate de diéthyle, le sébaçate de diisopropyle, le sébaçate de dibutyle ou le sébaçate de dioctyle, sans que cette liste ne soit limitative. On utilise de préférence un diester saturé en C₄-C₈ d'alkyle en C₆-C₁₀, plus préférentiellement l'adipate de dioctyle. Il a en effet été démontré que l'association de ce diester avec le monoester précité permettait d'éviter le gonflement de certains matériaux siliconés sans nuire aux autres propriétés recherchées pour ces matériaux. Le rapport massique du monoester en C₁₀-C₁₈ d'alkyle en Ci-C₈ au diester saturé en C₂-C₁₀ d'alkyle en C₃-C₁₂ peut notamment être compris entre 5:95 et 95:5, de préférence entre 40:60 et 60:40.

Avantageusement, le monoester en C₁₀-C₁₈ d'alkyle en C₁-C₈ et le diester saturé en C₂-C₁₀ d'alkyle en C₃-C₁₂, lorsqu'il est présent, représentent au moins 50% en poids, de préférence au moins 70% en poids, plus préférentiellement au moins 90% en poids, mieux, au moins 95% en poids, par rapport au poids du solvant selon l'invention. Mieux, le solvant utilisé selon l'invention ne renferme pas d'autre composé que le monoester en C₁₀-C₁₈ d'alkyle en C₁-C₈ et le diester saturé en C₂-C₁₀ d'alkyle en C₃-C₁₂, lorsqu'il est présent.

La concentration de la substance organique volatile dans le solvant peut être comprise entre 20 et 95% en poids, de préférence entre 30 et 90% en poids et plus préférentiellement entre 50 et 80% en poids.

Le matériau décrit précédemment est préparé suivant un procédé comprenant l'imprégnation du matériau polymérique solide avec une solution comprenant la substance organique volatile dans le solvant.

Dans une forme d'exécution préférée de l'invention, la substance organique volatile et le solvant décrits précédemment représentent au moins 50%, plus préférentiellement au moins 70%, mieux, au moins 90%, encore mieux, au moins 95% du poids de la solution. Plus particulièrement, la solution n'est constituée que du solvant et de la substance organique volatile. En variante, la solution d'infusion peut comprendre un ou plusieurs additifs choisis parmi les anti-oxydants, les antifongiques, les colorants, les stabilisants à la lumière, les absorbeurs UV, les agents de viscosité, les tensioactifs et leurs mélanges. Cette solution peut être préparée par simple mélange de ses constituants à température ambiante, généralement sous agitation.

L'imprégnation du matériau polymérique solide avec cette solution peut se faire par tout moyen connu de l'homme de l'art et notamment par trempage ou pulvérisation, préférentiellement par trempage du matériau dans un bain constitué de la solution. Un avantage de la présente invention est qu'un même bain peut être utilisé à plusieurs reprises, typiquement deux ou trois fois mais possiblement jusqu'à consommation totale de la solution, et permettre ainsi de préparer plusieurs articles à partir d'une solution unique. L'étape d'imprégnation est habituellement réalisée à température ambiante mais est également possible à toutes températures pour lesquelles la solution est à l'état liquide, permettant ainsi d'accélérer ou de ralentir l'infusion totale ou partielle de la solution dans le matériau.

Le matériau imprégné ainsi obtenu peut être utilisé pour parfumer ou désodoriser l'atmosphère, le corps ou le linge ou comme répulsif d'insectes ou insecticide, de préférence pour parfumer l'atmosphère. Lorsqu'il est utilisé comme produit parfumant ou désodorisant, ce matériau peut être mis en oeuvre dans tout type d'environnement, notamment dans des locaux à usage domestique, commercial ou industriel ou dans une voiture. En variante, il peut être introduit dans le tambour d'une machine à laver ou d'un sèche-linge, ou être utilisé en tant que composant d'un accessoire de mode (notamment de sac à main, bijou ou montre).

L'invention concerne donc également un article comprenant le matériau décrit précédemment, choisi parmi un produit parfumant ou désodorisant, un répulsif d'insectes ou un insecticide, de préférence un produit parfumant. Cet article peut présenter toute forme adaptée à ces utilisations et notamment se présenter sous forme d'anneau, de galet, de boule, de cube, de cylindre, de dôme ou de pyramide adaptés à être posés, suspendus ou portés. Il suffit pour ce faire que le matériau soit moulé sous la forme voulue avant imprégnation par la substance organique volatile. On notera qu'il est possible d'augmenter le taux de substance volatile incorporée dans l'article, soit en augmentant sa taille (à forme constante), soit en faisant varier sa forme de manière à augmenter sa surface, et ainsi de faire varier la durée d'utilisation de l'article en fonction de l'application envisagée.

### FIGURES

La Figure 1 illustre la variation, en fonction du temps, du taux d'infusion d'un parfum dans un matériau polymérique imprégné d'une solution de ce parfum dans différents solvants.
La Figure 2 illustre la variation, en fonction du temps, du taux de relargage d'un parfum infusé dans un matériau polymérique au moyen d'une solution contenant différents solvants.
La Figure 3 représente la variation, en fonction du temps, de l'intensité sensorielle dégagée par un parfum infusé dans un matériau polymérique au moyen d'une solution contenant différents solvants.
La Figure 4 représente le taux de gonflement d'un matériau siliconé imprégné d'une solution contenant un parfum dans différents solvants.
La Figure 5 illustre la variation, en fonction du temps, du taux d'infusion d'un parfum dans des matériaux siliconés de différentes duretés.
La Figure 6 illustre la variation, en fonction du temps, du taux de relargage d'un parfum infusé dans des matériaux siliconés de différentes duretés.

### EXEMPLES

L'invention sera mieux comprise à la lumière des exemples suivants, qui sont donnés à titre purement illustratif et n'ont pas pour but de limiter la portée de l'invention, définie par les revendications annexées.

### Exemple 1 : Influence du solvant sur le taux d'infusion

On a mesuré le taux d'infusion dans un matériau siliconé d'un parfum véhiculé dans différents solvants. Pour ce faire, des anneaux en élastomère de silicone identiques ont chacun été pesés puis immergés pendant différentes périodes de temps (1h, 4h, 18h et 24h) dans une solution contenant le même parfum dans un solvant différent, à savoir : l'éthanol (EtOH), l'oléate de méthyle, l'isopropylidène glycérol (Augeo MC), le myristate d'isopropyle (IPM), le 3-méthyl-3-méthoxybutanol (MMB), l'adipate de dioctyle (DOA) et un mélange 50:50 (p/p) de myristate d'isopropyle et d'adipate de dioctyle (DOA/IPM). La concentration en parfum dans la solution allait de 50% en poids (pour les solvants individuels) à 80% en poids (pour le mélange de solvants). L'anneau a été prélevé à l'issue de la période de temps considérée et pesé pour déterminer le pourcentage de parfum qu'il contenait.

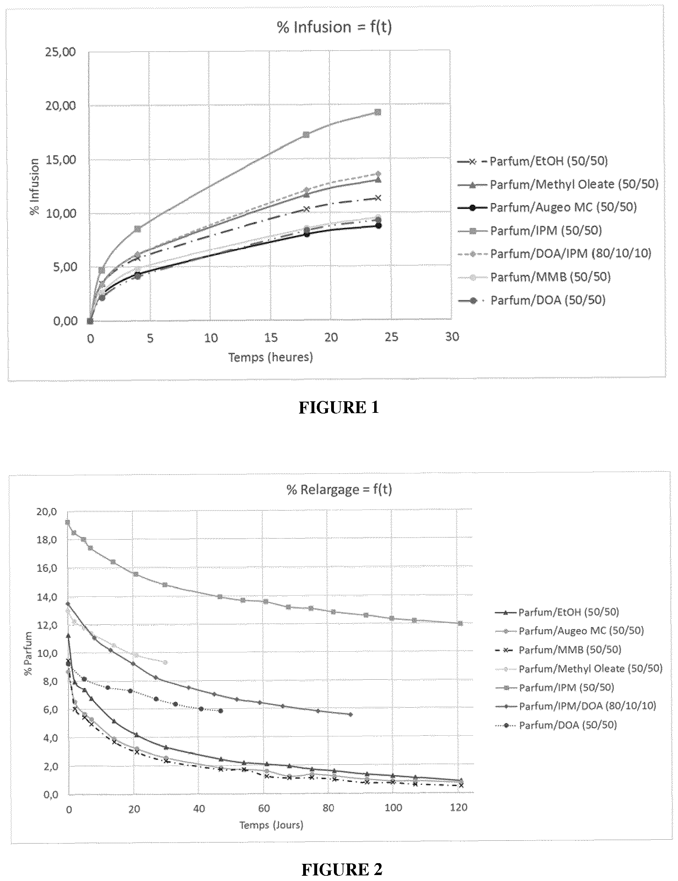

Les résultats de ces essais sont illustrés sur la Figure 1. Comme le montre cette Figure, le myristate d'isopropyle permet d'obtenir le taux d'infusion le plus important. Le taux d'infusion diminue lorsque ce solvant est mélangé à l'adipate de dioctyle, en restant toutefois supérieur à ce que permettent d'obtenir d'autres solvants tels que le 3-méthyl-3-méthoxybutanol, utilisé comme agent compatibilisant dans US-6,379,689 et les diesters d'alkyle tels que décrits dans FR 1 601 586.

### Exemple 2 : Influence du solvant sur le taux de relargage

On a mesuré le taux de relargage ou de diffusion d'un parfum véhiculé dans différents solvants et infusé dans le même matériau siliconé. Pour ce faire, des anneaux en élastomère de silicone identiques ont chacun été immergés pendant 24h dans une solution contenant le même parfum dans un solvant différent, à savoir : l'éthanol (EtOH), l'oléate de méthyle, l'isopropylidène glycérol (Augeo MC), le myristate d'isopropyle (IPM), le 3-méthyl-3-méthoxybutanol (MMB), l'adipate de dioctyle (DOA) et un mélange 50:50 (p/p) de myristate d'isopropyle et d'adipate de dioctyle (DOA/IPM). La concentration en parfum dans la solution allait de 50% en poids (pour les solvants individuels) à 80% en poids (pour le mélange de solvants). L'anneau a ensuite été pesé après différentes durées de conservation à température ambiante pour déterminer le pourcentage de parfum restant dans l'anneau.

Les résultats de ces essais sont illustrés sur la Figure 2. Comme le montre cette Figure, la cinétique de relargage du parfum dépend du solvant testé. Le myristate d'isopropyle permet une diffusion progressive du parfum qui reste présent dans l'anneau en quantité nettement supérieure, après 120 jours, à ce que permettent d'obtenir les autres solvants testés, en particulier le 3-méthyl-3-méthoxybutanol et l'adipate de dioctyle. On notera que l'essai utilisant l'oléate de méthyle a été interrompu en raison d'une exsudation du solvant.

Des essais complémentaires ont permis de montrer que la cinétique de relargage du parfum véhiculé dans le myristate d'isopropyle ne variait pas significativement lorsque la concentration du parfum dans le solvant variait de 30 à 90% en poids.

### Exemple 3 : Influence du solvant sur l'intensité sensorielle du parfum

On a mesuré l'intensité sensorielle d'un parfum véhiculé dans différents solvants et infusé dans le même matériau siliconé. Pour ce faire, des anneaux en élastomère de silicone identiques ont chacun été immergés pendant 24h dans une solution contenant le même parfum dans un solvant différent, à savoir : l'éthanol (EtOH), l'oléate de méthyle, l'isopropylidène glycérol (Augeo MC), le myristate d'isopropyle (IPM), le 3-méthyl-3-méthoxybutanol (MMB), l'adipate de dioctyle (DOA) et un mélange 50:50 (p/p) de myristate d'isopropyle et d'adipate de dioctyle (DOA/IPM). La concentration en parfum dans la solution allait de 50% en poids (pour les solvants individuels) à 80% en poids (pour le mélange de solvants). L'anneau a ensuite été conservé à température ambiante dans un placard pendant deux mois. Un panel d'experts entraînés a évalué l'intensité sensorielle du parfum après différentes durées de conservation. Celle-ci a été notée sur une échelle de 0 à 10 où 10 correspond à l'intensité sensorielle initiale du parfum.

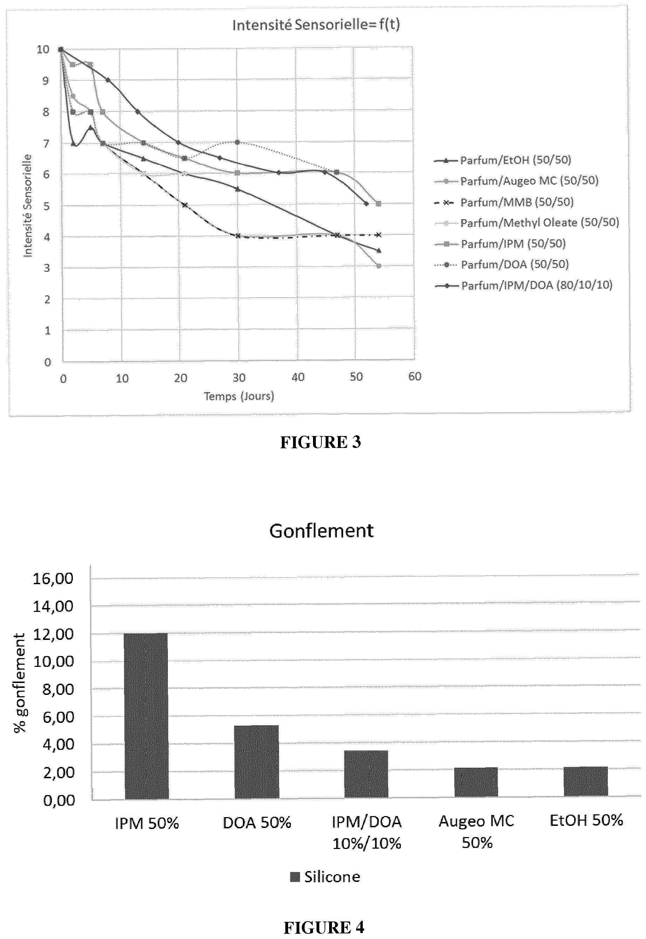

Les résultats de ces essais sont illustrés sur la Figure 3. Comme le montre cette Figure, l'intensité sensorielle du parfum en solution dans le myristate d'ispropyle, seul ou en mélange avec l'adipate de dioctyle, chute moins fortement qu'en présence des autres solvants (en particulier le 3-méthyl-3-méthoxybutanol) et diminue de manière plus linéaire qu'avec l'adipate de dioctyle seul.

### Exemple 4 : Influence du solvant sur le gonflement du matériau

On a mesuré le taux de gonflement d'un anneau en élastomère de silicone dans différents solvants, à savoir : l'éthanol (EtOH), l'isopropylidène glycérol (Augeo MC), le myristate d'isopropyle (IPM), l'adipate de dioctyle (DOA) et un mélange 50:50 (p/p) de myristate d'isopropyle et d'adipate de dioctyle (DOA/IPM). La concentration en parfum dans la solution allait de 50% en poids (pour les solvants individuels) à 80% en poids (pour le mélange de solvants). Il a été observé, comme il ressort de la Figure 4, que le gonflement du matériau était le plus important dans le myristate d'isopropyle et qu'il pouvait être significativement réduit en l'associant à l'adipate de dioctyle. Comme démontré dans les Exemples 1 à 3, le mélange IPM / DOA permet d'incorporer une quantité appréciable de parfum dans le matériau et présente de bonnes propriétés de diffusion du parfum, de sorte qu'il constitue un solvant de choix pour les matériaux à base d'élastomère de silicone.

### Exemple 5 : Influence de la dureté du matériau

On a évalué, de manière similaire à l'Exemple 1, le taux d'infusion d'un parfum véhiculé dans l'isopropyl myristate (20% p/p) dans différents élastomères de silicone, préparés à partir de résines de silicone ayant une dureté Shore A allant de 30 (MED-4930 de NuSil) à 80 (MED-4980 de NuSil).

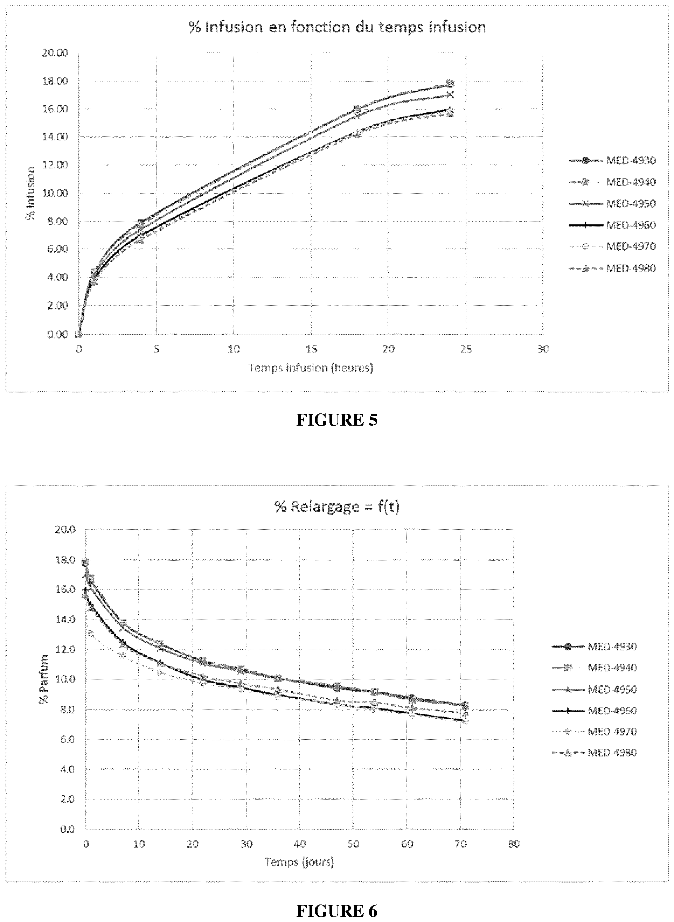

Les résultats de cet essai sont présentés à la Figure 5. Comme le montre cette figure, la dureté de l'élastomère a peu d'impact sur le taux d'infusion du parfum, les matériaux les plus durs étant légèrement moins chargés de parfum que les matériaux plus mous.

On a également mesuré la vitesse de relargage du parfum par ces différents matériaux, de manière similaire à l'Exemple 2. Comme illustré à la Figure 6, la dureté de l'élastomère a un impact non significatif sur les propriétés de relargage du parfum, dans la mesure où la pente de la courbe obtenue en fonction du temps varie peu.

## Revendications

1. Matériau polymérique solide imprégné d'une substance organique volatile et d'un solvant comprenant un monoester en C₁₀-C₁₈ d'alkyle en Ci-Cs, le matériau étant obtenu suivant un procédé comprenant les étapes suivantes :
(a) la solubilisation de la substance organique volatile dans ledit solvant, et
(b) l'imprégnation du matériau polymérique solide avec la solution ainsi obtenue.

2. Matériau selon la revendication 1, **caractérisé en ce que** la substance organique volatile est choisie parmi un parfum, un agent masqueur d'odeur et un insecticide, de préférence un parfum.

3. Matériau selon la revendication 1 ou 2, **caractérisé en ce que** le solvant comprend un monoester en C₁₂-C₁₆ d'alkyle en C₃-C₆, de préférence le myristate d'isopropyle.

4. Matériau selon l'une quelconque des revendications 1 à 3, **caractérisé en ce que** la concentration de la substance organique volatile dans le solvant est comprise entre 20 et 95% en poids, de préférence entre 30 et 90% en poids et plus préférentiellement entre 50 et 80% en poids.

5. Matériau selon l'une quelconque des revendications 1 à 4, **caractérisé en ce qu'**il comprend un élastomère de silicone.

6. Matériau selon la revendication 5, **caractérisé en ce que** le solvant renferme en outre un diester saturé en C₂-C₁₀ d'alkyle en C₃-C₁₂, de préférence un diester saturé en C₄-C₈ d'alkyle en C₆-C₁₀, plus préférentiellement l'adipate de dioctyle.

7. Matériau selon la revendication 6, **caractérisé en ce que** le rapport massique du monoester en C₁₀-C₁₈ d'alkyle en Ci-Cs au diester saturé en C₂-C₁₀ d'alkyle en C₃-C₁₂ est compris entre 5:95 et 95:5, de préférence entre 40:60 et 60:40.

8. Procédé de fabrication du matériau selon l'une quelconque des revendications 1 à 7, **caractérisé en ce qu'**il comprend les étapes suivantes :
(a) la solubilisation de la substance organique volatile dans le solvant, et
(b) l'imprégnation du matériau polymérique solide avec la solution ainsi obtenue.

9. Utilisation non thérapeutique du matériau selon l'une quelconque des revendications 1 à 7 pour parfumer ou désodoriser l'atmosphère, le corps ou le linge ou comme répulsif d'insectes ou insecticide, de préférence pour parfumer l'atmosphère.

10. Article comprenant le matériau selon l'une quelconque des revendications 1 à 7, **caractérisé en ce qu'**il est choisi parmi un produit parfumant ou désodorisant, un répulsif d'insectes ou un insecticide, de préférence un produit parfumant.

## Patentansprüche

1. Feststoffliches Polymermaterial, das mit einer flüchtigen organischen Substanz und mit einem Lösungsmittel durchtränkt ist, welches einen C₁-C₈-Alkyl-C₁₀-C₁₈-monoester umfasst, wobei das Material gemäß einem Verfahren erhalten wird, welches die folgenden Schritte umfasst:
(a) Inlösungbringen der flüchtigen organischen Substanz in dem Lösungsmittel, und
(b) Durchtränken des feststofflichen Polymermaterials mit der Lösung, welche auf diese Weise erhalten wurde.

2. Material nach Anspruch 1, **dadurch gekennzeichnet, dass** die flüchtige organische Substanz aus einem Duftstoff, einem geruchsmaskierenden Stoff und einem Insektizid, vorzugsweise einem Duftstoff, ausgewählt ist.

3. Material nach Anspruch 1 oder 2, **dadurch gekennzeichnet, dass** das Lösungsmittel einen C₃-C₆-Alkyl-C₁₂-C₁₆-monoester, vorzugsweise Isopropylmyristat, umfasst.

4. Material nach einem beliebigen der Ansprüche 1 bis 3, **dadurch gekennzeichnet, dass** die Konzentration der flüchtigen organischen Substanz in dem Lösungsmittel im Bereich von 20 bis 95 Gewichts-%, vorzugsweise 30 bis 90 Gewichts-%, und stärker bevorzugt 50 bis 80 Gewichts-%, liegt.

5. Material nach einem beliebigen der Ansprüche 1 bis 4, **dadurch gekennzeichnet, dass** es ein Silikonelastomer umfasst.

6. Material nach Anspruch 5, **dadurch gekennzeichnet, dass** das Lösungsmittel darüber hinaus einen gesättigten C₃-C₁₂-Alkyl-C₂-C₁₀-diester, vorzugsweise einen gesättigten C₆-C₁₀-Alkyl-C₄-C₈-diester, stärker bevorzugt Dioctyladipat, enthält.

7. Material nach Anspruch 6, **dadurch gekennzeichnet, dass** das Massenverhältnis zwischen dem C₁-C₈-Alkyl-C₁₀-C₁₈-monoester und dem gesättigten C₃-C₁₂-Alkyl-C₂-C₁₀-diester im Bereich von 5:95 und 95:5, vorzugsweise von 40:60 bis 60:40, liegt.

8. Verfahren zur Herstellung des Materials nach einem beliebigen der Ansprüche 1 bis 7, **dadurch gekennzeichnet, dass** es die folgenden Schritte umfasst:
(a) Inlösungbringen der flüchtigen organischen Substanz in dem Lösungsmittel, und
(b) Durchtränken des feststofflichen Polymermaterials mit der Lösung, welche auf diese Weise erhalten wurde.

9. Nicht-therapeutische Verwendung des Materials nach einem beliebigen der Ansprüche 1 bis 7, um die Raumluft, den Körper oder Wäsche zu parfümieren oder zu deodorieren, oder als Insektenrepulsivstoff oder Insektizid, vorzugsweise um die Raumluft zu parfümieren.

10. Gegenstand, welcher das Material nach einem beliebigen der Ansprüche 1 bis 7 umfasst, **dadurch gekennzeichnet, dass** er aus einem parfümierenden oder deodorierenden Produkt, einem insektenvergrämenden Mittel oder einem Insektizid ausgewählt ist, wobei es sich vorzugsweise um ein parfümierendes Produkt handelt.

## Claims

1. A solid polymeric material impregnated with a volatile organic substance and with a solvent comprising a C₁₀-C₁₈ monoester of a C₁-C₈ alkyl, wherein said material is prepared by a process comprising the following steps:
(a) solubilizing the volatile organic substance in said solvent, and
(b) impregnating the solid polymeric material with the solution thus obtained.

2. The material as claimed in claim 1, **characterized in that** the volatile organic substance is chosen from a fragrance, an odor-masking agent and an insecticide, preferably a fragrance.

3. The material as claimed in claim 1 or 2, **characterized in that** the solvent comprises a C₁₂-C₁₆ monoester of a C₃-C₆ alkyl, preferably isopropyl myristate.

4. The material as claimed in any one of claims 1 to 3, **characterized in that** the concentration of the volatile organic substance in the solvent is between 20 and 95% by weight, preferably between 30 and 90% by weight and more preferentially between 50 and 80% by weight.

5. The material as claimed in any one of claims 1 to 4, **characterized in that** it comprises a silicone elastomer.

6. The material as claimed in claim 5, **characterized in that** the solvent also contains a saturated C₂-C₁₀ diester of a C₃-C₁₂ alkyl, preferably a saturated C₄-C₈ diester of a C₆-C₁₀ alkyl, more preferentially dioctyl adipate.

7. The material as claimed in claim 6, **characterized in that** the mass ratio of the C₁₀-C₁₈ monoester of a C₁-C₈ alkyl to the saturated C₂-C₁₀ diester of a C₃-C₁₂ alkyl is between 5:95 and 95:5, preferably between 40:60 and 60:40.

8. A process for producing the material as claimed in any one of claims 1 to 7, **characterized in that** it comprises the following steps:
(a) solubilizing the volatile organic substance in the solvent, and
(b) impregnating the solid polymeric material with the solution thus obtained.

9. The use of the material as claimed in any one of claims 1 to 7, for fragrancing or deodorizing the atmosphere, the body or laundry or as an insect repellant or insecticide, preferably for fragrancing the atmosphere.

10. An article comprising the material as claimed in any one of claims 1 to 7, **characterized in that** it is chosen from a fragrancing or deodorizing product, an insect repellant or an insecticide, preferably a fragrancing product.
